# EUROPEAN PATENT APPLICATION

(11) **EP 4 180 042 A1**
(43) Date of publication of application: **17.05.2023**
(21) Application number: 22207087.2
(22) Date of filing: 14.11.2022
(51) Int. Cl.: A61K 31/519, A61K 9/20

(54) **A FILM COATED TABLET COMPRISING MICRONIZED TOFACITINIB**

(30) Priority: 15.11.2021 TR 202117729
(71) Applicant: SANOVEL ILAC SANAYI VE TICARET A.S., Istanbul 34460 (TR)
(72) Inventor: GULER, Tolga, Istanbul (TR); SUNEL, Fatih, Istanbul (TR); PEHLIVAN AKALIN, Nur, Istanbul (TR)
(74) Representative: Sevinç, Erkan

(57) **Abstract**

The present invention relates to a film coated tablet formulation comprising tofacitinib or pharmaceutically acceptable salts thereof wherein tofacitinib or pharmaceutically acceptable salts thereof has a d (0.5) particle size less than 20 µm. Further, the present invention also relates to a simple, rapid, cost effective, time-saving and industrially convenient process.

## Description

### Field of the Invention

The present invention relates to a film coated tablet formulation comprising tofacitinib or pharmaceutically acceptable salts thereof wherein tofacitinib or pharmaceutically acceptable salts thereof has a d (0.5) particle size less than 20 µm. Further, the present invention also relates to a simple, rapid, cost effective, time-saving and industrially convenient process.

### Background of the Invention

The chemical name of tofacitinib is 3-((3R,4R)-4-methyl-3-[methyl-(7H-pyrrolo[2,3-d]pyrimidin-4-yl)-amino]-piperidin-1-yl)-3-oxopropionitrile. It has the chemical formula C₁₆H₂₀N₆O and the following structural Formula I.

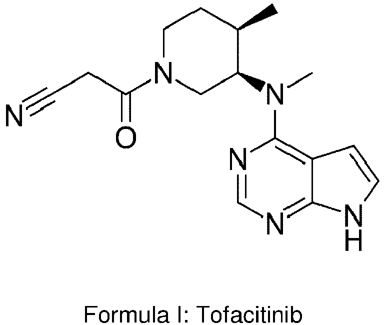

Tofacitinib is being developed as an immediate release tablet form with doses ranging from 5 mg to 10 mg administered BID (two times a day). Tofacitinib, as the citrate salt of tofacitinib, is approved in the US under the brand XELJANZ^{™}. XELJANZ^{®} is also available as a once-daily tablet called XELJANZ^{®} XR. XELJANZ^{®} XR is approved in the extended-release tablets form with 17.77 mg tofacitinib citrate (equivalent to 11 mg tofacitinib). Both XELJANZ^{®} and XELJANZ^{®} XR tablets are supplied for oral administration and are indicated for the treatment of rheumatoid arthritis.

Tofacitinib, as well as certain pharmaceutically acceptable salts thereof, is described in WO2001/042246, WO2002/096909, WO2003/048162, WO2012/135338, WO2013/090490 and US2015/0225406 describes crystalline forms and describes process for preparing certain other Tofacitinib salts.

U.S. Patent No. 6,956,041 provides various dosage forms of tofacitinib for oral, parenteral, buccal, or intranasal administration. It further mentions pharmaceutical compositions for oral administration, for example, tablets or capsules prepared by conventional means with pharmaceutically acceptable excipients.

Tofacitinib, a weakly basic compound, is classified as a BCS Class 3 compound (high solubility, low permeability). Also, tofacitinib is used small proportion that can lead to considerable problems during the manufacture of the composition with regard to the uniformity of the content of active agent in the individual composition units. Because of problems uniformity of the content, the active substance may interact with several excipients. It reflects that content uniformity play important role in the dissolution of the drug.

There still remains a need in the prior art to provide an improved pharmaceutical tablet formulation comprising tofacitinib or pharmaceutically acceptable salts thereof.

At this application, tofacitinib or pharmaceutically acceptable salts thereof has a d (0.5) particle size less than 20 µm in order to overcomes the described problems in prior art. So, this provides the desired solubility, content uniformity, compressibility of the tablet and permeability. Also, the film coated tablet has been developed by using standard techniques which is simple and cost-effective method.

### Detailed Description of the Invention

The main object of the present invention is to eliminate problems caused by tofacitinib and bringing additional advantages to the relevant prior art.

Another object of the present invention is to provide a film coated tablet comprising tofacitinib or pharmaceutically acceptable salts thereof having the desired dissolution profile, stability and content uniformity.

Another object of the present invention is to provide a film coated tablet having the desired compressibility and flowability.

Tofacitinib is used small proportion that can lead to considerable problems during the manufacture of the composition with regard to the uniformity of the content of active agent in the individual composition units. We have found that tofasitinib or pharmaceutically acceptable salts thereof having the following particle sizes is very important for formulation. Especially, it positively affects the dissolution properties and powder homogenization. The obtained tablets have the desired dissolution profile. The powder is more homogeneous. The content uniformity of the tablets obtained from the more homogeneous powder is more ideal. As used here in, 'particle size' means the cumulative volume size distrubition as tested by any conventionally accepted method such as the laser diffraction method (i.e. malvern analysis). The term d (0.1) means, the size at which 10% by volume of the particles are finer and d (0.5) means the size at which 50% by volume of the particles are finer and d (0.9) means the size at which %90 by volume of the particles are finer.

According to this embodiment of the present invention, the film coated tablet comprises tofasitinib or pharmaceutically acceptable salts thereof and at least one pharmaceutically acceptable excipient wherein tofasitinib or pharmaceutically acceptable salts thereof has a d (0.5) particle size less than 20 µm.

According to this embodiment of the present invention, tofacitinib or pharmaceutically acceptable salts thereof has a d (0.5) particle size less than 17 µm, preferably less than 12 µm.

According to this embodiment of the present invention, tofacitinib or pharmaceutically acceptable salts thereof has a d (0.5) particle size between 4 µm and 11 µm.

According to this embodiment of the present invention, tofacitinib or pharmaceutically acceptable salts thereof has a d (0.1) particle size less than 10 µm, preferably less than 5 µm.

According to this embodiment of the present invention, tofacitinib or pharmaceutically acceptable salts thereof has a d (0.9) particle size less than 35 µm, preferably less than 30 µm.

According to this embodiment of the present invention, tofacitinib or pharmaceutically acceptable salts thereof has a d (0.9) particle size between 22 µm and 30 µm.

According to this embodiment of the present invention, tofacitinib or pharmaceutically acceptable salts thereof has a d (0.5) particle size less than 20 µm, a d (0.1) particle size less than 10 µm, a d (0.9) particle size less than 35 µm. This provides the desired dissolution profile. Also, this helps to provide content uniformity of the tablets.

According to this embodiment of the present invention, the amount of tofacitinib or pharmaceutically acceptable salts is between 1.0% and 10.0%, preferably between 1.0% and 6.0% by weight in the total film coated tablet. Tofacitinib or pharmaceutically acceptable salts is used small proportion that can cause homogeneity problems. However, we have prevented this with the mentioned particle size and effective formulation and a process. According to this embodiment of the present invention, tofacitinib is present in the form of tofacitinib citrate.

In general terms, excipients provided in a formulation may positively or negatively influence the physicochemical and pharmacokinetic properties, e.g. the solubility, absorption, bioavailability of an active agent. For this reason, the excipients which accompany an active agent have to be selected in a careful and conscious manner while a formulation is developed. The formulations should have no physicochemical incompatibility between the active agents and the excipients.

According to this embodiment of the present invention, the film coated tablet comprises pharmaceutically acceptable excipients are selected from the group comprising fillers, disintegrants, lubricants, glidants or mixtures thereof.

Suitable fillers are selected from the group comprising microcrystalline cellulose, lactose anhydrous, lactose, lactose monohydrate, polyvinylpyrrolidone, ammonium alginate, calcium carbonate, calcium phosphate, calcium phosphate dehydrate, neutral pellets, calcium sulfate, cellulose, cellulose acetate, compressible sugar, dextrose, ethylcellulose, fructose, glyceryl palmitostearate, lactitol, mannitol, magnesium carbonate, magnesium oxide, maltodextrin, maltose, medium chain triglycerides, polydextrose, polymethacrylates, sodium alginate, sodium chloride, sorbitol, sucrose, sugar spheres, sulfobutylether beta-cyclodextrin, talc, tragacanth, trehalose, xylitol or mixtures thereof.

According to one embodiment of the present invention, the filler is microcrystalline cellulose or lactose anhydrous or mixtures thereof.

According to one embodiment of the present invention, the amount of fillers is between 65.0% and 95.0%, preferably between 75.0% and 93.0% by weight in the total film coated tablet.

According to one embodiment of the present invention, the amount of microcrystalline cellulose is between 45.0% and 70.0%, preferably between 50.0% and 65.0% by weight in the total film coated tablet.

According to one embodiment of the present invention, the amount of lactose anhydrous is between 20.0% and 35.0%, preferably between 25.0% and 32.0% by weight in the total film coated tablet.

Suitable disintegrants are selected from the group comprising croscarmellose sodium, crospovidone, low-substituted hydroxypropyl cellulose, carboxymethyl cellulose, sodium carboxymethyl cellulose, calcium carboxymethyl cellulose, sodium carboxymethyl starch, hydroxymethyl starch or mixtures thereof.

According to one embodiment of the present invention, the disintegrant is croscarmellose sodium.

According to one embodiment of the present invention, the amount of disintegrants is between 1.0% and 7.0%, preferably between 2.0% and 5.0% by weight in the total film coated tablet.

Suitable lubricants are selected from the group comprising magnesium stearate, calcium stearate, sodium stearyl fumarate, potassium stearate, stearic acid, high melting point waxes, sodium chloride, sodium benzoate, sodium acetate, sodium oleate, polyethylene glycols or mixtures thereof.

According to one embodiment of the present invention, the lubricant is magnesium stearate.

According to one embodiment of the present invention, the amount of lubricants is between 0.1% and 2.0% by weight in the total film coated tablet.

Suitable glidants are selected from group comprising colloidal silicon dioxide, water soluble excipient, hydrophilic polymers, silicon dioxide or mixtures thereof.

According to one embodiment of the present invention, the glidant is colloidal silicon dioxide. It enhances the compressibility by increasing the hardness of the tablet and provides the desired flowability of the tablet powders.

According to one embodiment of the present invention, the amount of glidants is between 0.1% and 2.0% by weight in the total film coated tablet.

According to one embodiment of the present invention, the film coated tablet comprises tofasitinib or pharmaceutically acceptable salts thereof and at least one pharmaceutically acceptable excipient wherein tofasitinib or pharmaceutically acceptable salts thereof has a d (0.5) particle size less than 20 µm and glidant is colloidal silicon dioxide.

According to one embodiment of the present invention, the tablet comprises;
- Tofacitinib citrate
- Microcrystalline cellulose (PH 112)
- Lactose (Supertab 24AN_anhydrous)
- Croscarmellose sodium
- Colloidal silicone dioxide
- Magnesium Stearate

According to another embodiment of the present invention, the film coated tablet comprises;
- 1.0-10.0% by weight of tofacitinib or pharmaceutically acceptable salts thereof
- 45.0-70.0% by weight of microcrystalline cellulose
- 20.0 - 35.0% by weight of lactose
- 1.0% - 7.0% by weight of croscarmellose sodium
- 0.1% - 2.0% by weight of colloidal silicone dioxide
- 0.1% - 2.0% by weight of magnesium stearate
- 1.0% - 5.0% by weight of film coating agent in the total tablet formulation.

According to this embodiment of the invention, the tablet is prepared with direct compression. Direct compression is the simplest form of oral dosage production and low-cost method, once extremely common and again becoming more popular because of its simplicity and cost efficiency. Furthermore, we found surprisingly that direct compression improves the physical and chemical stability of tablets as compared to wet granulation.

According to this embodiment of the present invention, a process for preparing a film coated tablet comprise the following steps:
a) Mixing tofacitinib citrate, croscarmellose sodium, colloidal silicone dioxide and a half of microcrystalline cellulose,
b) Sieving the mixture at step (a),
c) Adding lactose and the remaining part of microcrystalline cellulose and then mixing,
d) Sieving the mixture at step (c),
e) Adding magnesium stearate and then mixing,
f) Compressing to form of tablets,
g) Coating tablets with film coating.

### Example 1:

| **Ingredients** | **Amount (% by weight of the total formulation)** |
|---|---|
| Micronized tofacitinib citrate | 1.0 - 10.0 |
| Microcrystalline cellulose (PH 112) | 45.0 - 70.0 |
| Lactose (Supertab 24AN_anhydrous) | 20.0 - 35.0 |
| Croscarmellose sodium | 1.0 - 7.0 |
| Colloidal silicone dioxide | 0.1 - 2.0 |
| Magnesium Stearate | 0.1 - 2.0 |
| Film coating | 1.0 - 5.0 |
| **TOTAL** | **100** |

### Example 2:

| **Ingredients** | **Amount (% by weight of the total formulation)** |
|---|---|
| Tofacitinib citrate | 3.9 |
| Microcrystalline cellulose (PH 112) | 58.46 |
| Lactose (Supertab 24AN_anhydrous) | 29.5 |
| Croscarmellose sodium | 2.9 |
| Colloidal silicone dioxide | 0.5 |
| Magnesium Stearate | 1.0 |
| Film coating | 3.8 |
| **TOTAL** | **100** |

A process for example 1 or 2;
a) Mixing tofacitinib citrate, croscarmellose sodium, colloidal silicone dioxide and a half of microcrystalline cellulose,
b) Sieving the mixture at step (a),
c) Adding lactose and the remaining part of microcrystalline cellulose and then mixing,
d) Sieving the mixture at step (c),
e) Adding magnesium stearate and then mixing,
f) Compressing to form of tablets,
g) Coating tablets with film coating.

## Claims

1. A film coated tablet comprising tofasitinib or pharmaceutically acceptable salts thereof and at least one pharmaceutically acceptable excipient wherein tofasitinib or pharmaceutically acceptable salts thereof has a d (0.5) particle size less than 20 µm.

2. The film coated tablet according to claim 1, wherein tofacitinib or pharmaceutically acceptable salts thereof has a d (0.1) particle size less than 10 µm, preferably less than 5 µm.

3. The film coated tablet according to claim 1, wherein tofacitinib or pharmaceutically acceptable salts thereof has a d (0.9) particle size less than 35 µm, preferably less than 30 µm.

4. The film coated tablet according to claim 1, wherein tofacitinib or pharmaceutically acceptable salts thereof has a d (0.5) particle size less than 20 µm, a d (0.1) particle size less than 10 µm, a d (0.9) particle size less than 35 µm.

5. The film coated tablet according to claim 1, wherein the film coated tablet comprises pharmaceutically acceptable excipients are selected from the group comprising fillers, disintegrants, lubricants, glidants or mixtures thereof.

6. The film coated tablet according to claim 5, wherein the filler is microcrystalline cellulose or lactose anhydrous or mixtures thereof.

7. The film coated tablet according to claim 5, wherein the disintegrant is croscarmellose sodium.

8. The film coated tablet according to claim 5, wherein the lubricant is magnesium stearate.

9. The film coated tablet according to claim 5, wherein glidants are selected from group comprising colloidal silicon dioxide, water soluble excipient, hydrophilic polymers, silicon dioxide or mixtures thereof.

10. The film coated tablet according to claim 5, wherein the glidant is colloidal silicon dioxide.

11. The film coated tablet according to claim 10, wherein the amount of glidants is between 0.1% and 2.0% by weight in the total film coated tablet.

12. The film coated tablet according to claim 1, wherein, the tablet comprises;
- Tofacitinib citrate
- Microcrystalline cellulose (PH 112)
- Lactose (Supertab 24AN_anhydrous)
- Croscarmellose sodium
- Colloidal silicone dioxide
- Magnesium Stearate

13. The film coated tablet according to claim 1, wherein the film coated tablet comprises;
- 1.0-10.0% by weight of tofacitinib or pharmaceutically acceptable salts thereof
- 45.0-70.0% by weight of microcrystalline cellulose
- 20.0 - 35.0% by weight of lactose
- 1.0% - 7.0% by weight of croscarmellose sodium
- 0.1% - 2.0% by weight of colloidal silicone dioxide
- 0.1% - 2.0% by weight of magnesium stearate
- 1.0% - 5.0% by weight of film coating agent in the total tablet formulation.

14. A process for preparing a film coated tablet comprise the following steps:
a) Mixing tofacitinib citrate, croscarmellose sodium, colloidal silicone dioxide and a half of microcrystalline cellulose,
b) Sieving the mixture at step (a),
c) Adding lactose and the remaining part of microcrystalline cellulose and then mixing,
d) Sieving the mixture at step (c),
e) Adding magnesium stearate and then mixing,
f) Compressing to form of tablets,
g) Coating tablets with film coating.
